## Europäisches Patentamt
## European Patent Office
## Office européen des brevets

(19)

(11) Veröffentlichungsnummer: **0 072 915**
**B1**

(12) **EUROPÄISCHE PATENTSCHRIFT**

(45) Veröffentlichungstag der Patentschrift:
**21.05.86**

(51) Int. Cl.⁴: **C 07 C 118/02**, C 07 C 119/048

(21) Anmeldenummer: **82106345.0**

(22) Anmeldetag: **15.07.82**

(54) Verfahren zur Herstellung von 2,4,6-Triisocyanato-toluol.

(30) Priorität: **20.08.81 DE 3132923**

(43) Veröffentlichungstag der Anmeldung:
**02.03.83 Patentblatt 83/9**

(45) Bekanntmachung des Hinweises auf die Patenterteilung:
**21.05.86 Patentblatt 86/21**

(84) Benannte Vertragsstaaten:
**AT BE CH DE FR GB IT LI NL SE**

(56) Entgegenhaltungen:
**GB - A - 631 007**
**GB - A - 631 025**

(73) Patentinhaber: **BASF Aktiengesellschaft,
Carl-Bosch-Strasse 38, D-6700 Ludwigshafen (DE)**

(72) Erfinder: **Schuster, Ludwig, Dr., Weinheimer Strasse 44,
D-6703 Limburgerhof (DE)**

LIBER, STOCKHOLM 1986

**Beschreibung**

Nach Angaben der britischen Patentschriften 631 007 und 631 025 wird 2,4,6-Triisocyanato-toluol durch Phosgenierung einer Suspension aus 2,4,6-Triamino-toluolhydrochlorid und einem inerten Lösungsmittel bei Temperaturen über 90°C hergestellt. Hierbei soll das inerte Lösungsmittel einen Siedepunkt kleiner als 160°C besitzen. Im einzelnen werden als Lösungsmittel genannt: Kohlenwasserstoffe, wie Toluol, Xylol oder Ligroin, halogenierte Kohlenwasserstoffe, wie Tetrachlorethan, Monochlorbenzol oder o-Chlortoluol, Ether, wie Anisol oder Dioxan oder Gemische eines dieser Lösungsmittel mit z.B. Ethylendichlorid oder Benzol. Nach dieser Arbeitsweise beträgt die Ausbeute an 2,4,6-Triisocyanato-toluol ungefähr 73 % der Theorie.

Aufgabe der vorliegenden Erfindung war es 2,4,6-Triiso-cyanato-toluol nach einem technisch relativ einfachen Verfahren in guten Ausbeuten herzustellen.

Diese Aufgabe konnte überraschenderweise durch die Verwendung von o-Dichlorbenzol oder höhersiedenden Carbonsäureestern als Lösungsmittel gelöst werden.

Gegenstand der vorliegenden Erfindung ist somit ein Verfahren zur Herstellung von 2,4,6-Triisocyanato-toluol durch Umsetzung von 2,4,6-Triamino-toluolhydrochlorid mit Phosgen in Gegenwart eines inerten Lösungsmittels, das dadurch gekennzeichnet ist, daß man als Lösungsmittel o-Dichlorbenzol oder Mono- und/oder Dicarbonsäureester mit Siedepunkten von 165° bis 250°C verwendet.

Da die Abtrennung von 2,4,6-Triisocyanato-toluol durch Destillation aus hochsiedenden Lösungsmitteln erschwert wird, konnte nicht erwartet werden, daß die Verwendung von o-Dichlorbenzol (Siedepunkt: 182°C) oder Mono- und/oder Dicarbonsäureestern mit Siedepunkten von 165° bis 250°C zu einer deutlichen Ausbeutesteigerung führt. Darüber hinaus ist ein direkter Einfluß des Lösungsmittel auf die Reaktion feststellbar. Hervorzuheben ist ferner die verbesserte Löslichkeit des 2,4,6-Triamino-toluolhydrochlorids in den Carbonsäurestern.

Von besonderer Bedeutung ist auch die feine Verteilung des 2,4,6-Triamino-toluolhydrochlorids in den genannten Lösungsmitteln während der Phosgenierung. Diese wird durch Ausfällen des Hydrochlorids in Alkohol mit Chlorwasserstoff und nachfolgender Abtrennung des Alkohols unter gleichzeitiger Zuführung der erfindungsgemäß verwendbaren Lösungsmittel bei nahezu konstanter 2,4,6-Triamino-toluolhydrochloridkonzentration erzielt. Auf besondere Vorrichtungen, die die Aminhydrochloridherstellung in dünnen Plüssigkeitsschichten oder -filmen ermöglichen, kann daher ebenso verzichtet werden, wie auf eine Naßvermahlung des gebildeten Aminhydrochlorids zur Gewinnung einer feinteiligen Suspension.

Zur Herstellung der 2,4,6-Triaminotoluolhydrochlorid-Suspension werden 2 bis 15 Gew.-Teile, vorzugsweise 3 bis 10 Gew.-Teile 2,4,6-Triamino-toluol in 85 bis 98 Gew.-Teilen vorzugsweise 90 bis 97 Gew.-Teilen eines Alkohols oder einer Alkoholmischung mit 1 bis 3 C-Atomen im Alkylrest, wie z.B. Ethanol, n-Propanol, Isooropanol und vorzugsweise Methanol, gelöst. In die Lösung wird unter Rühren bei Temperaturen von 0 bis 80°C, vorzugsweise 10 bis 60°C 0,8 bis 1,1 Mol, vorzugsweise ungefähr 1 Mol Chlorwasserstoff pro $NH_2$-Gruppe des Triaminotoluols eingeleitet. Zur Erhöhung der Löslichkeit des Chlorwasserstoffs im Alkohol kann die Aminhydrochloridbildung auch unter erhöhtem Druck, beispielsweise von 1 bis 3 bar durchgeführt werden.

Nach beendeter 2,4,6-Triaminotoluolhydrochloridbildung wird die alkoholische Suspension unter Rühren erhitzt und der Alkohol abdestilliert. Gleichzeitig wird der Suspension in gleichem Maße o-Dichlorbenzol oder Mono- und/oder Dicarbonsäureester einverleibt, so daß die 2,4,6-Triamino-toluolhydrochloridkonzentration, die ungefähr 4 bis 40 Gew.%, vorzugsweise 8 bis 25 Gew.%, bezogen auf das Gesamtgewicht der Suspension, beträgt nahezu konstant bleibt. Gegen Ende der Alkoholdestillation, die üblicherweise in 0,5 bis 3 Stunden, vorzugsweise 0,5 bis 1,5 Stunden durchgeführt wird, wird die Temperatur bis zum Siedepunkt des Lösungsmittels erhöht und gegebenenfalls bis zu 10 Gew.% des zugeführten Lösungsmittels unter gegebenenfalls gleichzeitig weiterer o-Dichlorbenzol, Mono- und/oder Dicarbonsäureesterzugabe abdestilliert. Auf diese Weise erreicht man nicht nur einen vollkommenen Alkohol-Lösungsmittel-austausch, sondern man erhält eine wasser- und alkoholfreie Suspension von feinkristallinem 2,4,6-Triamino-toluolhydrochlorid in o-Dichlorbenzol oder Mono- und/oder Dicarbonsäureester.

Die erhaltene Suspension von 2,4,6-Triamino-toluolhydrochlorid in o-Dichlorbenzol oder Mono- und/oder Dicarbonaäureester wird danach bei 100 bis 150°C, vorzugsweise 120° bis 140°C mit i bis 3 Mol, vorzugsweise 1,1 bis 1,5 Mol Phosgen pro $NH_2$.HCl-Gruppe zur Reaktion gebracht und das intermediär gebildete Carbamidsäurechlorid in 2,4,6-Triisocyanato-toluol gespalten. Das gasförmige Phosgen wird hierbei der Suspension mit einer solchen Geschwindigkeit zugeführt, daß die austretenden Gase überwiegend aus Chlorwasserstoff bestehen. Die Phosgenierungs- und Spaltungszeit beträgt durchschnittlich 36 bis 48 Stunden.

Sobald die suspendierten Salzteilchen verschwunden sind, wird das o-Dichlorbenzol oder der Mono- und/oder Dicarbonsäureester, vorzugsweise unter vermindertem Druck, beispielsweise von 20 bis 40 mbar abdestilliert. Gegebenenfalls kann es auch vorteilhaft sein, den Chlorwasserstoff und überschüssiges Phosgen mit Hilfe von Stickstoff oder einem anderen Inertgas aus der Isocyanatlösung auszutreiben, bevor das Lösungsmittel abdestilliert wird.

Die erfindungsgemäß verwendbaren Mono- und/oder Dicarbonsäureester besitzen Siedepunkte von 165° bis 250°C und werden zweckmäßigerweise aus aromatischen Monocarbonsäuren oder aliohatischen Dicarbonsäuren mit 2 bis 6 C-Atomen und Alkoholen mit 1 bis 4 C-Atomen im Alkylrest hergestellt. Beispielhaft genannt seien Benzoesäuremethylester, Oxalsäurediethylester und Adipinsäuredimethylester.

In Carbonsäureestern mit höheren Siedepunkten, beispielsweise Phthalsäuredimethylester, kann das 2,4,6-Triiso-cyanato-toluol ebenfalls in sehr guten Ausbeuten hergestellt werden. Nachteilig an dieser Verfahrensvariante ist jedoch, daß das Reaktionsgemisch praktisch nicht mehr durch eine einfache Destillation trennbar ist.

Das erhaltene rohe 2,4,6-Triisocyanato-toluol wird anschließend durch Destillation unter vermindertem Druck gereinigt. Der Siedepunkt beträgt bei 0,2 mbar 125-130°C.

Die destillative Reinigung wird zweckmäßigerweise in Gegenwart eines hochsiedenden polaren Verdünnungsmittels durchgeführt. Als Verdünnungsmittel haben sich besonders Mono- und/oder Dicarbonsäureester mit Siedepunkten über 300°C, vorzugsweise über 320°C bewährt, so daß diese bevorzugt Anwendung finden. Im einzelnen seien beispielhaft genannt: Mono- und Diester von aromatischen Carbonsäuren und Alkoholen und Phenolen mit 1 bis 12 C-Atomen, wie z.B. Diisooctyl-, Diethylphthalat, Trioctyltrimellitat, Diisobutylterephthalat, Diethylenglykoldibenzoat und Phenylbenzoat. Vorzugsweise verwendet werden Diester der Phthalsäure mit Alkoholen mit 8 bis 12 C-Atomen oder entsprechenden Alkoholmischungen.

Durch die Mitverwendung des Verdünnungsmittels, das in Mengen von 20 bis 30 Gew.-Teilen pro Gew.-Teil rohes 2,4,6-Triisocyanato-toluol eingesetzt wird, kann die Bildung von sehr harten, in allen Lösungsmitteln vollkommen unlöslichen pechartigen Rückständen in den Destillationsgefäßen, die sich nur sehr mühsam mechanisch entfernen lassen, vermieden werden. Auf die beschriebene Weise enthält man bis zum Ende der Destillation einen dünnflüssigen Sumof, aus dem das 2,4,6-Triisocyanato-toluol sogar mit Hilfe eines Dünnschichtverdampfers abdestilliert werden kann.

Nach dem erfindungsgemäßen Verfahren kann 2,4,6-Triisocyanato-toluol in Ausbeuten größer als 85 % der Theorie hergestellt werden. Das Triisocyanat wird vorzugsweise zur Herstellung von Polyurethanen, insbesondere Polyurethanklebstoff, -Dichtungsmitteln, -Lacken und -Überzügen verwendet.

**Beispiel 1**

In einem Rührgefäß werden 68,5 g (0,5 Mol) reines Triaminotoluol in 1,3 1 Methanol gelöst und 61 g (1,4 Mol) Chlorwasserstoff unter Rühren eingeleitet. Anschließend destilliert man das Methanol ab und ersetzt während des Abdestillierens das verdampfte Methanol durch o-Dichlorbenzol. Gegen Ende destilliert man noch etwa 0,10 1 Dichlorbenzol ab, so daß man eine Suspension von 123,5 g Hydrochlorid in 1,2 1 Dichlorbenzol erhält. Nun leitet man bei einer Temperatur von 110 bis 130°C einen Strom von Phosgen so ein, daß das austretende Gas überwiegend aus Chlorwasserstoff besteht. Wenn alle suspendierten Salzteilchen verschwunden sind, destilliert man das Dichlorbenzol unter einem Druck von 20 mbar ab. Anschließend setzt man 36 g eines Diesters aus Phthalsäure und einer Alkoholmischung mit 9 bis 11 C-Atomen zu und destilliert unter einem Druck von 0,2 mbar das Triisocyanat ab; Sdp. 125 bis 130°C. Man erhält 96,4 g; dies sind 89,7 % d. Th. Der Rückstand ist in der Hitze dünnflüssig und läßt sich mit üblichen Lösungsmitteln entfernen.

**Beispiele 2 bis 4 und Vergleichsbeispiele**

Man verfährt analog den Angaben des Beispiels 1, verwendet jedoch anstelle von o-Dichlorbenzol andere Lösungsmittel.

Die eingesetzten Lösungsmittel und die damit erzielten Ausbeuten sind in der folgenden Tabelle zusammengefaßt.

| Beispiele | Lösungsmittel | Ausbeute an 2,4,6--Triisocyanato-toluol [g] | [% d. Theori] |
|---|---|---|---|
| 2 | Benzoesäuremethylester | 99,7 | 92,7 |
| 3 | Oxalsäurediethylester | 94,5 | 87,9 |
| 4 | Adipinsäuredimethyl-ester | 92,0 | 86,0 |
| Vergleichs-beispiele | | | |
| | Xylol | 78,5 | 73 |
| | Phthalsäuredimethyl-ester (Reaktionsmischung nicht mehr durch Destillation trennbar) | 98,1 | 91,3 |

**Patentansprüche**

1. Verfahren zur Herstellung von 2,4,6-Triisocyanato-toluol durch Umsetzung von 2,4,6-Triamino-toluolhydrochlorid mit Phosgen in Gegenwart eines inerten Lösungsmittels, dadurch gekennzeichnet, daß man als Lösungsmittel o-Dichlorbenzol oder Mono- und/oder Dicarbonsäureester mit Siedepunkten von 165° bis 250°C verwendet.

2. Verfahren nach Anspruch 1, dadurch gekennzeichnet, daß man als Lösungsmittel o-Dichlorbenzol verwendet.

3. Verfahren nach Anspruch 1, dadurch gekennzeichnet, daß man als Monocarbonsäureester Benzoesäuremethylester verwendet.

4. Verfahren nach Anspruch 1, dadurch gekennzeichnet, daß man als Dicarbonsäureester Oxalsäurediethyl ester oder Adipinsäuredimethylester verwendet.

5. Verfahren nach Anspruch 1, dadurch gekennzeichnet, daß man

a) 2,4,6-Triamino-toluol mit Chlorwasserstoff in Gegenwart von Alkohol in 2,4,6-Triamino-toluolhydrochlorid überführt,

b) den Alkohol beim jeweiligen Siedepunkt aus der erhaltenen Suspension abdestilliert und in gleichem Maße der Suspension o-Dichlorbenzol oder Mono- und/oder Dicarbonsäureester einverleibt, so die 2,4,6-Triamino-toluolhydrochloridkonzentration ungefähr konstant bleibt,

c) das 2,4,6-Triamino-toluolhydrochlorid in Gegenwart von o-Dichlorbenzol oder Mono- und/oder Dicarbonsäureester phosgeniert und das intermediär gebildete Carbamidsäurechlorid in 2,4,6-Triisocyanato-toluol spaltet und

d) das o-Dichlorbenzol oder die Mono- und/oder Dicarbonsäureester abtrennt.

6. Verfahren nach Anspruch 5, dadurch gekennzeichnet, daß man das 2,4,6-Triamino-toluol mit Chlorwasserstoff in Gegenwart von Methanol bei Temperaturen von 10 bis 60°C in das Hydrochlorid überführt.

7. Verfahren nach Anspruch 5, dadurch gekennzeichnet, daß die 2,4,6-Triamino-toluolhydrochloridkonzentration (b) in der Suspension 4 bis 40 Gew.% beträgt.

8. Verfahren nach Anspruch 5, dadurch gekennzeichnet, daß man das erhaltene rohe 2,4,6-Triisocyanato-toluol in Gegenwart eines Mono- und/oder Dicarbonsäureesters mit einem Siedepunkt von über 300°C als Verdünnungsmittel destilliert.

## Claims

1. A process for the preparation of 2,4,6-triisocyanatotoluene by reacting 2,4,6-triaminotoluene hydrochloride with phosgene in the presence of an inert solvent, wherein odichlorobenzene or a monocarboxylic and/or dicarboxylic acid ester having a boiling point of from 165 to 250°C is used as solvent.

2. A process as claimed in claim 1, wherein o-dichlorobenzene is used as solvent.

3. A process as claimed in claim 1, wherein methyl benzoate is used as monocarboxylic acid ester.

4. A process as claimed in claim 1, wherein diethyl oxalate or dimethyl adipate is used as dicarboxylic acid ester.

5. A process as claimed in claim 1, wherein

a) 2,4,6-triaminotoluene is converted with hydrogen chloride, in the presence of an alcohol, into 2,4,6-triaminotoluene hydrochloride,

b) the alcohol is distilled off from the resulting suspension at the boiling point concerned, and o-dichlorobenzene or the monocarboxylic and/or dicarboxylic acid ester is incorporated into the suspension at such a rate that the concentration of 2,4,6-triaminotoluene hydrochloride remains approximately constant,

c) the 2,4,6-triaminotoluene hydrochloride is phosgenated in the presence of o-dichlorobenzene or the monocarboxylic andtor dicarboxylic acid ester, and the carbamic acid chloride formed as intermediate is cleaved into 2,4,6-triisocyanatotoluene, and

d) the o-dichlorobenzene or the monocarboxylic and/or dicarboxylic acid ester is separated off.

6. A process as claimed in claim 5, wherein the 2,4,6-tri-aminotoluene is converted with hydrogen chloride, in the presence of methanol, into the hydrochloride at a temperature of from 10 to 60°C.

7. A process as claimed in claim 5, wherein the concentration of 2,4,6-triaminotoluene hydrochloride (b) in the suspension is from 4 to 40% by weight.

8. A process as claimed in claim 5, wherein the resulting crude 2,4,6-triisocyanatotoluene is distilled in the presence of a monocarboxylic and/or dicarboxylic acid ester having a boiling point above 300°C as diluent.

## Revendications

1.- Procédé de préparation de 2,4,6-triisocyanato-toluène par réaction de chlorhydrate de 2,4,6-triamino-toluène avec du phosgène, en présence d'un solvant inerte, caractérisé par le fait que l'on utilise, comme solvant, du o-dichlorobenzène ou un ester d'acide mono- et/ou -dicarboxylique d'un point d'ébullition de 165° à 250°C.

2.- Procédé selon la revendication 1, caractérisé par le fait que l'on utilise, comme solvant, du o-dichlorobenzène.

3.- Procédé selon la revendication 1, caractérisé par le fait que l'on utilise, comme ester d'acide monocarboxylique, du benzoate de méthyle.

4.- Procédé selon la revendication 1, caractérisé par le fait que l'on utilise, comme ester d'acide dicarboxylique, de l'oxalate de diethyle ou de l'adipinate de diméthyle.

5.- Procédé selon la revendication i, caractérisé par le fait que

a) on fait passer le 2,4,6-triamino-toluène, avec de l'acide chlorhydrique, en présence d'alcool, en chlorhydrate de 2,4,6-triamino-toluène,

b) de la suspension obtenue, on sépare par distillation, l'alcool au point d'ébullition respectif et on incorpore, dans la même mesure, à la suspension, du o-dichlorobenzène ou de l'ester d'acide mono-et/ou dicarboxylique, de manière que la concentration en chlorhydrate de 2,4,6-triamino-toluène reste à peu près constante,

c) on phosgénise le chlorhydrate de 2,4,6-triamino-toluène, en présence de o-dichlorobenzène ou d'ester d'acide mono- et/ou dicarboxylique, et on scinde en 2,4,6-triisocyanato-toluène le chlorure d'acide carbarmidique intermédiaire forme,

d) on sépare le o-dichlorobenzène ou l'ester d'acide mono- et/ou dicarboxylique.

6.- Procédé selon la revendication 5, caractérisé par le fait que l'on fait passer le 2,4,6 triamino-toluène en chlorhydrate, avec de l'acide chlorhydrique, en presence de méthanol, à des températures de 10 à 60°C.

7.- Procédé selon la revendication 5, caractérisé par le fait que la concentration du chlorhydrate de 2,4,6 triamino-toluène (b) dans la suspension est de 4 à 40 % en poids.

8.- Procédé selon la revendication 5, caractérisé par le fait que l'on distille le 2,4,6 triisocyanato-toluène brut obtenu, en présence d'un ester d'acide mono- et/ou dicarboxylique d'un point d'ébullition de plus de 300°C, comme diluant.